# EUROPEAN PATENT APPLICATION

(11) **EP 3 112 344 A1**
(43) Date of publication of application: **04.01.2017**
(21) Application number: 15174514.8
(22) Date of filing: 30.06.2015
(51) Int. Cl.: C07C 257/02, C07C 327/44, C07D 257/04

(54) **METHOD FOR PREPARATION OF CERTAIN THIOACETAMIDES**

(71) Applicant: Lonza Ltd, 3930 Visp (CH)
(72) Inventor: ZARAGOZA DOERWALD, Florencio, 3930 Visp (CH)

(57) **Abstract**

The invention discloses a method for the preparation of certain thioacetamides with sulfur chlorides starting from certain methylketones, and their use in the preparation of certain 1,5 disubstituted tetrazoles, which allows the preparation of said tetrazoles from readily available compounds such as acetophenone and similar compounds.

## Description

The invention discloses a method for the preparation of certain thioacetamides with sulfur chlorides starting from certain methylketones, and their use in the preparation of certain 1,5 disubstituted tetrazoles, which allows the preparation of said tetrazoles from readily available compounds such as acetophenone and similar compounds.

### BACKGROUND OF THE INVENTION

Picarbutrazox with CAS 500207-04-5 is a fungicide useful as plant protecting agent. A key intermediate for the preparation of picarbutrazox is 1-methyl-5-benzoyltetrazole. EP 2546236 A1 discloses the steps from 1-methyl-5-benzoyltetrazole to picarbutrazox.

EP 2407461 A1 discloses a method for preparation of 1-methyl-5-benzoyltetrazole from methyl 2-oxo-2-phenylacetate, via intermediate conversion of N-methyl-2-oxo-2-phenylacetamide into an imidoyl chloride. Methyl 2-oxo-2-phenylacetate, however, is an expensive intermediate. Moreover, amides are unreactive compounds, and the conversion of amides into imidoyl chlorides can sometimes require elevated reaction temperatures, causing the formation of byproducts and low yields of the required imidoyl chloride. The reaction times disclosed in EP 2407461 A1 are rather long. The reaction temperatures are rather high

WO 2011/110651 A1 discloses a method for preparation of 1-methyl-5-benzoyltetrazole starting from benzoylchloride. The method uses methyl isocyanide. Methyl isocyanide is a substance that is problematic to use in large scale production due its high toxicity, high volatility, and high reactivity. The production of methyl isocyanide usually causes the formation of large amounts of phosphorus-containing waste, which is expensive to dispose of.

There was a need for a method that does not use methyl isocyanide or methyl 2-oxo-2-phenylacetate.

Unexpectedly a method for preparation of certain 1,5 disubstituted tetrazols was found starting from certain thioacetamides, which ultimately allows the preparation of these certain 1,5 disubstituted tetrazoles also from readily available compounds such as acetophenone and similar compounds.

The method does not use methyl isocyanide or methyl 2-oxo-2-phenylacetate.

In the following text,
- ambient: pressure maens usually 1 bar, depending on the weather;
- halide: means F , Cl⁻, Br or I⁻, preferably Cl⁻, Br or I⁻, more preferably Cl⁻ or Br ;
- halogen: means F, Cl, Br or I, preferably Cl, Br or I;
if not otherwise stated.

### SUMMARY OF THE INVENTION

Subject of the invention is a method for the preparation of compound of formula (II); 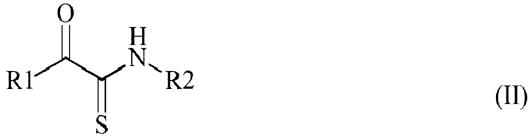 compound of formula (II) is prepared in two steps, a step ST1 and a step ST2;
ST1 comprises a reaction REAC1 of compound of formula (I) with a compound SULFCHLO; SULFCHLO is S₂Cl₂, SCl₂, or a mixture thereof; 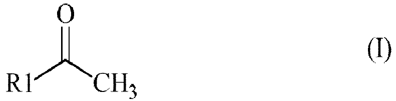 REAC1 results in a reaction product REACPROD1;
ST2 comprises a reaction REAC2 of REACPROD1 with R2-NH₂;
REAC2 results in compound of formula (II);
wherein
- R1: is selected from the group consisting of
tertiary C₄₋₁₂ alkyl,
phenyl, the phenyl being unsubstituted or substituted by 1, 2, 3, 4 or 5 substituents selected from the group consisting of halogen, nitro, cyano, trifluoromethyl, C₁₋₈ alkyl, and C₁₋₈ alkoxy, and
5 or 6 membered aromatic heterocycle with 1, 2 or 3 heteroatoms selected from the group consisting of N, O and S, the 5 or 6 membered aromatic heterocycle being unsubstituted or substituted by 1, 2 or 3 substituents selected from the group consisting of halogen, nitro, cyano, trifluoromethyl, C₁₋₈ alkyl, and C₁₋₈ alkoxy;
- R2: is selected from the group consisting of
C₁₋₁₂ alkyl, the C₁₋₁₂ alkyl being unsubstituted or substituted by 1, 2 or 3 substituents selected from the group consisting of phenyl or 5 or 6 membered aromatic heterocycle with 1, 2 or 3 heteroatoms selected from the group consisting of N, O and S,
C₃₋₆ cycloalkyl,
phenyl, the phenyl being unsubstituted or substituted by 1, 2, 3, 4 or 5 substituents selected from the group consisting of halogen, nitro, cyano, trifluoromethyl, C₁₋₈ alkyl, and C₁₋₈ alkoxy, and
5 or 6 membered aromatic heterocycle with 1, 2 or 3 heteroatoms selected from the group consisting of N, O and S, the 5 or 6 membered aromatic heterocycle being unsubstituted or substituted by 1, 2 or 3 substituents selected from the group consisting of halogen, nitro, cyano, trifluoromethyl, C₁₋₈ alkyl, and C₁₋₈ alkoxy.

### DETAILED DESCRIPTION OF THE INVENTION

Preferably, R1 is selected from the group consisting of
tertiary C₄₋₁₂ alkyl,
phenyl, the phenyl being unsubstituted or substituted by 1, 2 or 3 substituents selected from the group consisting of halogen, nitro, cyano, trifluoromethyl, C₁₋₆ alkyl, and C₁₋₆ alkoxy, and
5 or 6 membered aromatic heterocycle with 1, 2 or 3 heteroatoms selected from the group consisting of N, O and S, the 5 or 6 membered aromatic heterocycle being unsubstituted or substituted by 1, 2 or 3 substituents selected from the group
consisting of halogen, nitro, cyano, trifluoromethyl, C₁₋₆ alkyl, and C₁₋₆ alkoxy; more preferably, R1 is selected from the group consisting of
tert-butyl,
phenyl, the phenyl being unsubstituted or substituted by 1, 2 or 3 substituents selected from the group consisting of halogen, nitro, trifluoromethyl, C₁₋₆ alkyl, and methoxy, and
5 or 6 membered aromatic heterocycle with 1, 2 or 3 heteroatoms selected from the group consisting of N, O and S, the 5 or 6 membered aromatic heterocycle being unsubstituted or substituted by 1, 2 or 3 substituents selected from the group
consisting of halogen, nitro, trifluoromethyl, C₁₋₆ alkyl, and methoxy;
even more preferably, R1 is selected from the group consisting of
tert-butyl,
phenyl, the phenyl being unsubstituted or substituted by 1 or 2 substituents selected from the group consisting of halogen, nitro, trifluoromethyl, C₁₋₆ alkyl, and methoxy, and
5 or 6 membered aromatic heterocycle with 1, 2 or 3 heteroatoms selected from the group consisting of N and S, the 5 or 6 membered aromatic heterocycle being unsubstituted or substituted by 1 or 2 substituents selected from the group consisting of halogen, nitro, trifluoromethyl, C₁₋₆ alkyl and methoxy.

Preferably, R2 is selected from the group consisting of
C₁₋₆ alkyl, the C₁₋₆ alkyl being unsubstituted or substituted by 1 substituent selected from the group consisting of phenyl or 5 or 6 membered aromatic heterocycle with 1, 2 or 3 heteroatoms selected from the group consisting of N, O and S,
C₃₋₆ cycloalkyl,
phenyl, the phenyl being unsubstituted or substituted by 1, 2 or 3 substituents selected from the group consisting of halogen, nitro, cyano, trifluoromethyl, C₁₋₆ alkyl, and C₁₋₆ alkoxy, and
5 or 6 membered aromatic heterocycle with 1, 2 or 3 heteroatoms selected from the group consisting of N, O and S, the 5 or 6 membered aromatic heterocycle being unsubstituted or substituted by 1, 2 or 3 substituents selected from the group
consisting of halogen, nitro, cyano, trifluoromethyl, C₁₋₆ alkyl, and C₁₋₆ alkoxy;
more preferably, R2 is selected from the group consisting of
methyl, ethyl, n-propyl, iso-propyl, butyl and pentyl, the methyl, ethyl, n-propyl, iso-propyl, butyl and pentyl being unsubstituted or substituted by 1 substituent selected from the group consisting of phenyl or 5 or 6 membered aromatic heterocycle with 1, 2 or 3 heteroatoms selected from the group consisting of N, O and S,
C₅₋₆ cycloalkyl, phenyl, the phenyl being unsubstituted or substituted by 1, 2 or 3 substituents selected from the group consisting of halogen, nitro, trifluoromethyl, C₁₋₆ alkyl, and methoxy, and
5 or 6 membered aromatic heterocycle with 1, 2 or 3 heteroatoms selected from the group consisting of N, O and S, the 5 or 6 membered aromatic heterocycle being unsubstituted or substituted by 1, 2 or 3 substituents selected from the group
consisting of halogen, nitro, trifluoromethyl, C₁₋₆ alkyl, and methoxy;
even more preferably, R2 is selected from the group consisting of
methyl, ethyl, n-propyl and iso-propyl, the methyl, ethyl, n-propyl and iso-propyl being unsubstituted or substituted by 1 substituent selected from the group consisting of phenyl or 5 or 6 membered aromatic heterocycle with 1, 2 or 3 heteroatoms selected from the group consisting of N, O and S,
C₅₋₆ cycloalkyl,
phenyl, the phenyl being unsubstituted or substituted by 1 or 2 substituents selected from
the group consisting of halogen, nitro, trifluoromethyl, C₁₋₆ alkyl, and methoxy, and
5 or 6 membered aromatic heterocycle with 1, 2 or 3 heteroatoms selected from the group consisting of N, O and S, the 5 or 6 membered aromatic heterocycle being unsubstituted or substituted by 1 or 2 substituents selected from the group consisting of halogen, nitro, trifluoromethyl, C₁₋₆ alkyl, and methoxy;

Preferably, the molar amount of R2-NH₂ in REAC2 is from 1 to 20 times, more preferably from 1 to 15 times, and even more preferably from 1 to 12 times, based on the molar amount of compound of formula (I).

Preferably, REAC2 is done at a temperature of from -40 °C to 100 °C, more preferably from -20 °C to 80 °C, even more preferably from from -10 °C to 50 °C.

Preferably, REAC2 is done at a pressure of from ambient pressure to 10 bar, more preferably from ambient pressure to 5 bar, even more preferably from ambient pressure to 2 bar.

Preferably, the reaction time of REAC2 is from 5 min to 48 h, more preferably from 20 min to 24 h, even more preferably from 30 min to 18 h, especially from 30 min to 10 h.

REAC2 can be done neat or in a solvent.

If REAC2 is done in a solvent, it is done preferably in a solvent SOLV2, SOLV2 is selected from the group consisting of benzene, toluene, xylene, chlorobenzene, pyridine, 3-picoline, 2-methyl-5-ethylpyridine, chloroform, dichloromethane, THF, water, and mixtures thereof;
more preferably, SOLV2 is selected from the group consisting of benzene, toluene, xylene, chlorobenzene, pyridine, 3-picoline, 2-methyl-5-ethylpyridine, dichloromethane, THF, water, and mixtures thereof.

Preferably, the weight of SOLV2 is from 0.01 to 50 times, more preferably from 0.05 to 20 times, even more preferably from 0.1 to 10 times, of the weight of compound of formula (I).

REAC2 can be done in the presence of a base. If REAC2 is done in the presence of a base, then the base is a compound BAS2, BAS2 is selected from the group consisting of sodium carbonate, sodium bicarbonate, potassium carbonate, sodium hydroxide, potassium hydroxide, calcium hydroxide, and mixtures therof;
more preferably, BAS2 is selected from the group consisting of sodium carbonate, potassium carbonate, sodium hydroxide, calcium hydroxide, and mixtures therof.

Preferably, the molar amount of BAS2 is from 1 to 10 times, more preferably from 1 to 5 times, and even more preferably from 1 to 3 times, based on the molar amount of compound of formula (I).

Compound of formula (II) can be isolated after REAC2 by any conventional method, for instance by extraction, by distillation under reduced pressure, or by crystallization. Preferably, any volatile byproduct is distilled off, and the residue is purified by crystallization, or used without further purification.

Preferably, the molar amount of SULFCHLO in REAC 1 is from 2 to 40 times, more preferably from 2 to 20 times, and even more preferably from 2 to 15 times, based on the molar amount of compound of formula (I).

It is known that S₂Cl₂, also known as disulfur dichloride or even referred to as sulfur monochloride, decomposes upon contact with water, e.g. moisture of air, into HCl, SO₂, and sulfur. It is also known that S₂Cl₂ decomposes upon heating into SCl₂, also known as sulfur dichloride, and into other sulfur chlorides. Moreover, S₂Cl₂ is prepared by chlorination of sulfur, and, depending on the precise reaction conditions and purification methods used, commercially available S₂Cl₂ may contain variable amounts of chlorine and SCl₂. Therefore SULFCHLO, that is used in REAC1, is preferably used in form of a compound COMPSULFCHLO, COMPSULFCHLO may have various qualities, various impurity contents and various impurity profiles. Therefore COMPSULFCHLO may contain e.g. up to 5%, 10%, 20%, 30% or even up to 50%, the % being % by weight based on the total weight of COMPSULFCHLO, of sulfur dichloride, chlorine, hydrogen chloride, SO₂, sulfur, or of mixtures of said impurities.

Therefore preferably, COMPSULFCHLO contains 50 to 100%, more preferably 70 to 100%, even more preferably 80 to 100%, especially 90 to 100%, more especially 95 top 100%, of SULFCHLO, the % being % by weight based on the total weight of COMPSULFCHLO.

S₂Cl₂ and SCl₂ are equivalent reagents for REAC2. S₂Cl₂ is commercially available. Preferably SULFCHLO is S₂Cl₂.

Preferably, REAC 1 is done at a temperature of from -20 °C to 80 °C, more preferably from -10 °C to 60 °C, even more preferably from from -5 °C to 40 °C.

The temperature of REAC 1 can be varied during the course of the reaction, so that REAC 1 is done with a temperature profile.

Preferably, REAC2 is done at a pressure of from ambient pressure to 100 bar, more preferably of from ambient pressure to 75 bar, even more preferably of from ambient pressure to 50 bar, especially of from ambient pressure to 30 bar, more especially of from ambient pressure to 25 bar, even more especially of from ambient pressure to 20 bar.

The pressure of REAC1 can be adjusted according to the chosen temperature of REAC1 and the boiling point of SULFCHLO.

Preferably, the reaction time of REAC1 is from 10 min to 72 h, more preferably from 30 min to 48 h, even more preferably from 1 h to 24 h.

REAC 1 can be done neat or in a solvent.

If REAC1 is done in a solvent, it is done preferably in a solvent SOLV1, SOLV1 is selected from the group consisting of benzene, chlorobenzene, toluene, dichloromethane, chloroform, acetonitrile, and mixtures thereof;
more preferably, SOLV1 is selected from the group consisting of benzene, chlorobenzene, toluene, dichloromethane, chloroform, and mixtures thereof.

Preferably, the weight of SOLV1 is from 0.01 to 20 times, more preferably from 0.05 to 10 times, even more preferably from 0.1 to 5 times, of the weight of compound of formula (I).

REAC 1 can be done in the presence of a catalyst. If REAC1 1 is done in the presence of a catalyst, then the catalyst is a compound CAT1, CAT1 is selected from the group consisting of pyridine, 2-picoline, 3-picoline, 4-picoline, 5-ethyl-2-methylpyridine, 4-(dimethylamino)pyridine, 1,4-diazabicyclo[2.2.2]octane, triethylamine, tributylamine, N-methylmorpholine, N-ethyldiisopropylamine, N,N-dimethylaniline, and mixtures thereof;
more preferably, CAT1 is selected from the group consisting of pyridine, 3-picoline, 5-ethyl-2-methylpyridine, 4-(dimethylamino)pyridine, 1,4-diazabicyclo[2.2.2]octane, triethylamine, tributylamine, N-methylmorpholine, N-ethyldiisopropylamine, N,N-dimethylaniline, and mixtures thereof;
even more preferably, CAT1 is selected from the group consisting of pyridine, 3-picoline, 5-ethyl-2-methylpyridine, 4-(dimethylamino)pyridine, 1,4-diazabicyclo[2.2.2]octane, triethylamine, tributylamine, N,N-dimethylaniline, and mixtures thereof.

Preferably, the molar amount of CAT1 is from 0.0001 to 0.6 times, more preferably from 0.0001 to 0.4 times, and even more preferably from 0.0001 to 0.3 times, based on the molar amount of compound of formula (I).

REACPROD1 can be isolated after REAC1 by any conventional method, for instance by extraction, by distillation under reduced pressure, or by crystallization. Preferably, any volatile byproduct is distilled off, and the residue is used without further purification.

Further subject of the invention is a method for the preparation of compound of formula (IV); 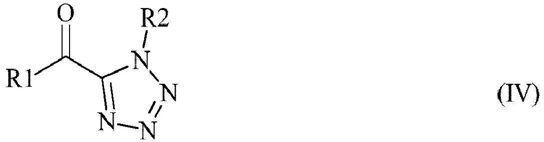 the method comprises four steps, ST1, ST2, a step ST3 and a step ST4;

ST4 comprises a reaction REAC4 of a compound of formula (III) with a compound AZID; 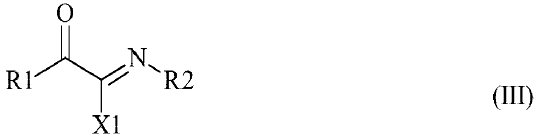 AZID is selected from the group consisting of alkali metal azide, alkali earth metal azide, [N(R10)(R11)(R12)R13]⁺ [N₃]⁻ and guanidinium azide;
R10, R11, R12 and R13 are identical or different and independently from each other selected from the group consisting of H and C₁₋₈ alkyl;
X1 is selected from the group consisting of Cl, Br, S-CH₃, S-CH₂-CH₃, S-CH₂-Phenyl, S-C(O)-C(CH₃)₃, and SO₃H;
REAC4 results in compound of formula (IV);
compound of formula (III) is prepared in ST3;
ST3 comprises a reaction REAC3 of a compound of formula (II) with a compound COMPREAC3;
COMPREAC3 is selected from the group consisting of thionyl chloride, COCl₂, diphosgene, triphosgene, POCl₃, PCl₃, PCl₅, POBr₃, PBr₃, PBr₅, S₂Cl₂, SCl₂, pivaloyl chloride, methyl chloride, methyl bromide, methyl iodide, ethyl chloride, ethyl bromide, ethyl iodide, benzyl chloride, benzyl bromide, dimethyl sulfate, diethyl sulfate, trimethylphosphate, triethylphosphate, methyl methanesulfonate, methyl benzenesulfonate, methyl tosylate, ethyl methanesulfonate, ethyl benzenesulfonate, ethyl tosylate, hydrogen peroxide, C₁₋₄ alkyl hydro peroxide, C₁₋₆ alkaloyl peroxide, perbenzoic acid, 3-chloro-perbenzoic acid, alkali metal persulfate salt, Cl₂, Br₂, and I₂;
REAC3 results in compound of formula (III);
compound of formula (II) is prepared in the two steps ST1 and ST2;
with ST1, ST2, R1 and R2 as defined herein, also with all their embodiments.

Preferably, AZID is selected from the group consisting of alkali metal azide, and [N(R10)(R11)(R12)R13]⁺ [N₃]⁻.

More preferably, AZID is selected from the group consisting of alkali metal azide, and [N(R10)(R11)(R12)R13]⁺ [N₃]⁻; and
R10 is C₁₋₄ alkyl; and
R11, R12 and R13 are identical or different and independently from each other selected from the group consisting of H and C₁₋₄ alkyl.

Even more preferably, AZID is selected from the group consisting of sodium azide, tetrabutylammonium azide, and triethylammonium azide.

Preferably, the molar amount of AZID is from 1 to 20 times, more preferably from 1 to 15 times, and even more preferably from 1 to 10 times, based on the molar amount of compound of formula (III).

Preferably, REAC4 is done at a temperature of from -60 °C to 100 °C, more preferably from -30 °C to 60 °C, even more preferably from from -10 °C to 40 °C.

Preferably, REAC4 is done at a pressure of from ambient pressure to 10 bar, more preferably from ambient pressure to 5 bar, even more preferably from ambient pressure to 2 bar.

Preferably, the reaction time of REAC4 is from 5 min to 24 h, more preferably from 15 min to 12 h, even more preferably from 30 min to 8 h. Preferably, REAC4 is done in a solvent SOLV4; SOLV4 is selected from the group consisting of water, acetone, acetonitrile, ethanol, methanol, ethylene glycol, benzene, toluene, chlorobenzene, N,N-dimethylformamide, NMP, THF, dioxane, ethyl acetate, butyl acetate, and mixtures thereof;
more preferably, SOLV4 is selected from the group consisting of water, acetone, ethanol, methanol, benzene, toluene, chlorobenzene, THF, dioxane, ethyl acetate, butyl acetate, and mixtures thereof;
even more preferably, SOLV4 is selected from the group consisting of water, benzene, toluene, chlorobenzene, ethyl acetate, butyl acetate, and mixtures thereof.

Preferably, the weight of SOLV4 is from 0.01 to 50 times, more preferably from 0.05 to 30 times, even more preferably from 0.1 to 20 times, of the weight of compound of formula (III).

REAC4 can be done in the presence of a compound PTCS4, PTCS4 is a compound of formula [PTCS4];

[N(R20)(R21)(R22)R23]⁺ [X2]⁻ [PTCS4]

R20, R21, R22 and R23 are identical or different and independently from each other selected from the group consisting of H, C₁₋₂₀ alkyl, phenyl and benzyl;
[X2]⁻ is selected from the group consisting of halide and hydrogensulfate.

More preferably, PTCS4 is selected from the group consisting of tetrabutylammonium halide, tetrabutylammonium hydrogensulfate, cetyltrimethylammonium chloride and bromide, and benzyltributylammonium chloride and bromide;
even more preferably, PTCS4 is selected from the group consisting of tetrabutylammonium chloride and bromide, tetrabutylammonium hydrogensulfate, cetyltrimethylammonium chloride, and benzyltributylammonium chloride.

Preferably, the molar amount of PTCS4 is from 0.001 to 1.0 times, more preferably from 0.005 to 0.8 times, and even more preferably from 0.01 to 0.5 times, based on the molar amount of compound of formula (III).

In one possible embodiment, REAC4 is done by mixing compound of formula (III) dissolved in SOLV4, SOLV4 except water, with AZID dissolved in water, thereby the reaction mixture in REAC4 has two liquid phases. In this embodiment PTCS4 can be used.

In another embodiment REAC4 is done by mixing compound of formula (III) dissolved in SOLV4, SOLV4 except water, with AZID in solid form. Also in this embodiment PTCS4 can be used.

The method of instant invention allows the use of PTCS4, but also works without the use of PTCS4.

Therefore in one embodiment, no PTCS4 is used in REAC4.

In another embodiment, no Bu₃N-CH₂-Ph or a chloride thereof is used.

In another embodiment, no PTCS4 and no Bu₃N-CH₂-Ph and no chloride of Bu₃N-CH₂-Ph is used.

In another embodiment, no phase transfer catalyst is used in REAC4.

When REAC4 is conducted in the presence of an aqueous phase, the pH of said aqueous phase can be adjusted by addition of a buffer. Preferably, the pH of the aqueous phase of REAC4 is between 0 and 14, more preferably between 2 and 10, and even more preferably between 3 and 8.

Preferably, when a buffer is used in REAC4, a buffer BUFF4 is used, BUFF4 is selected from the group consisting of acetate buffer, phosphate buffer, carbonate buffer, sulfate buffer, and mixtures thereof;
more preferably, BUFF4 is selected from the group consisting of acetate buffer, phosphate buffer and sulfate buffer.

Preferably, the molar amount of BUFF4 is from 0.01 to 20 times, more preferably from 0.05 to 6 times, and even more preferably from 0.1 to 3 times, based on the molar amount of compound of formula (III).

Compound of formula (IV) can be isolated after REAC4 by any conventional method, for instance by extraction and by crystallization. Preferably, any volatile byproduct is distilled off, and the residue is purified or used without further purification.

Preferably, COMPREAC3 is selected from the group consisting of thionyl chloride, COCl₂, diphosgene, triphosgene, POCl₃, PCl₃, PCl₅, S₂Cl₂, SCl₂, pivaloyl chloride, methyl chloride, methyl bromide, methyl iodide, ethyl chloride, ethyl bromide, ethyl iodide, benzyl chloride, benzyl bromide, dimethyl sulfate, diethyl sulfate, trimethylphosphate, triethylphosphate, methyl methanesulfonate, methyl benzenesulfonate, methyl tosylate, ethyl methanesulfonate, ethyl benzenesulfonate, ethyl tosylate, hydrogen peroxide, C₁₋₄ alkyl hydro peroxide, C₁₋₆ alkaloyl peroxide, perbenzoic acid, 3-chloro-perbenzoic acid, sodium persulfate salt, potassium persulfate salt, Cl₂, Br₂, and I₂;
more preferably, COMPREAC3 is selected from the group consisting of thionyl chloride, COCl₂, triphosgene, POCl₃, PCl₃, PCl₅, S₂Cl₂, methyl chloride, methyl bromide, methyl iodide, dimethyl sulfate, trimethylphosphate, methyl methanesulfonate, methyl benzenesulfonate, methyl tosylate, hydrogen peroxide, C₁₋₄ alkyl hydro peroxide, C₁₋₆ alkaloyl peroxide, perbenzoic acid, 3-chloro-perbenzoic acid, potassium persulfate salt, Cl₂, Br₂, and I₂;
even more preferably, COMPREAC3 is selected from the group consisting of thionyl chloride, COCl₂, triphosgene, POCl₃, S₂Cl₂, methyl chloride, dimethyl sulfate, trimethylphosphate, methyl benzenesulfonate, methyl tosylate, hydrogen peroxide, tert-butylhydroperoxide, peracetic acid, 3-chloro-perbenzoic acid, potassium persulfate salt, Cl₂, and Br₂.

Preferably, X1 is selected from the group consisting of Cl, S-CH₃, S-CH₂-CH₃, S-CH₂-Phenyl, S-C(O)-C(CH₃)₃, and SO₃H;
more preferably, X1 is selected from the group consisting of Cl, S-CH₃, and SO₃H.

Preferably, the molar amount of COMPREAC3 is from 1 to 50 times, more preferably from 1 to 30 times, and even more preferably from 1 to 20 times, based on the molar amount of compound of formula (II).

Preferably, REAC3 is done at a temperature of from -30 °C to 120 °C, more preferably from -10 °C to 90 °C, even more preferably from 0 °C to 60 °C, especially from 0 °C to 50 °C,
more especially from 0 °C to 40 °C, even more especially from 0 °C to 30 °C. Preferably, REAC3 is done at a pressure of from ambient pressure to 10 bar, more preferably from ambient pressure to 5 bar, even more preferably from ambient pressure to 2 bar.

Preferably, the reaction time of REAC3 is from 5 min to 24 h, more preferably from 10 min to 12 h, even more preferably from 30 min to 10 h, especially from 30 min to 8 h, more especially from 30 min to 7 h, even more especially from 30 min to 6 h, in particular from 30 min to 5 h, more in particular from 30 min to 4 h, even more in particular from 30 min to 3 h.

REAC3 can be done neat or in a solvent.

If REAC3 is done in a solvent, it is done preferably in is done in a solvent SOLV3, SOLV3 is selected from the group consisting of water, pyridine, 3-picoline, acetone, acetonitrile, benzene, toluene, chlorobenzene, N,N-dimethylformamide, NMP, THF, dioxane, ethyl acetate, butyl acetate, and mixtures thereof;
more preferably, SOLV3 is selected from the group consisting of water, pyridine, 3-picoline, acetone, benzene, toluene, chlorobenzene, THF, dioxane, ethyl acetate, butyl acetate, and mixtures thereof;
even more preferably, SOLV3 is selected from the group consisting of water, pyridine, 3-picoline, benzene, toluene, chlorobenzene, THF, dioxane, and mixtures thereof.

Preferably, the weight of SOLV3 is from 0.01 to 50 times, more preferably from 0.05 to 30 times, even more preferably from 0.1 to 20 times, of the weight of compound of formula (II).

Especially,
R1 is phenyl; and
R2 is methyl;
more especially,
SULFCHLO is S₂Cl₂;
R1 is phenyl; and
R2 is methyl;
even more especially,
SULFCHLO is S₂Cl₂;
COMPREAC3 is selected from the group consisting of thionyl chloride, COCl₂, hydrogen peroxide, S₂Cl₂ and Cl₂;
X1 is Cl or SO₃H;
AZID is sodium azide;
R1 is phenyl; and
R2 is methyl;
in particular,
SULFCHLO is S₂Cl₂;
COMPREAC3 is thionyl chloride;
X1 is Cl;
AZID is sodium azide;
R1 is phenyl;
R2 is methyl.

Compound of formula (III) can be isolated after REAC3 by any conventional method, for instance by extraction, distillation under reduced pressure, or by crystallization. Preferably, any volatile byproduct is distilled off, and the residue is used without further purification.

Preferred embodiments of compound of formula (I), compound of formula (II), compound of formula (III) and compound of formula (IV) are compound of formula (I-1), compound of formula (II-1), compound of formula (III-1) and compound of formula (IV-1).

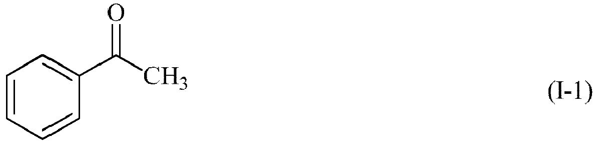

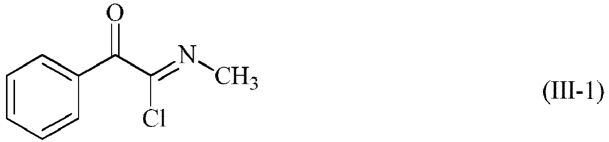

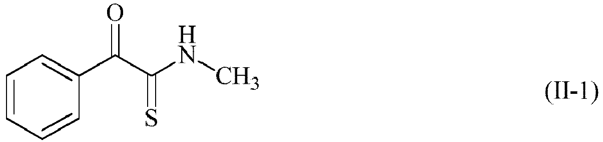

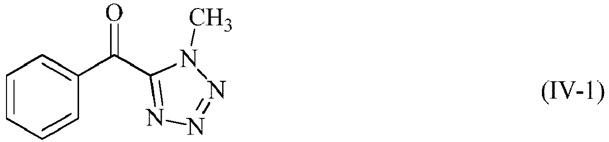

Further subject of the invention is a method for the preparation of compound of formula (VII), 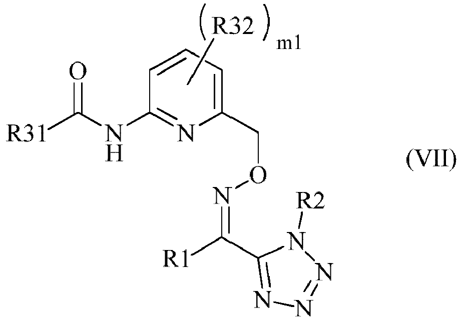 wherein the method comprises the two steps ST1 and ST2;
- R31: is selected from the group consisting of linear, branched and cyclic C₁₋₁₀ alkyl and linear, branched and cyclic C₁₋₁₀ alkoxy, wherein the linear, branched and cyclic C₁₋₁₀ alkyl and the linear, branched and cyclic C₁₋₁₀ alkoxy can be unsubstituted or substituted by 1, 2 or 3 substituents selected from the group consisting of F, Cl, Br, C₁₋₄ alkoxy, C₁₋₄ alkoxy substituted by F, Cl, difluoro, dichloro, bromo or dibromo, carbomethoxy, carboethoxy and OH;
- R32: is selected from the group consisting of F, Cl, Br, CN, NO₂, OH, SH, C(O)H, COOH, N(R50)R51,
linear, branched and cyclic C₁₋₁₀ alkyl, wherein the linear, branched and cyclic C₁₋₁₀ alkyl can be unsubstituted or substituted by 1, 2 or 3 substituents selected from the group consisting of F, Cl, Br, C₁₋₄ alkoxy, C₁₋₄ alkoxy substituted by F, Cl, difluoro, dichloro, bromo or dibromo, carbomethoxy, carboethoxy and OH,
phenyl, naphthyl, wherein the phenyl and the napthyl are unsubstituted or substituted by 1, 2 or 3 substituents selected from the group consisting of C₁₋₄ alkyl, F, Cl, trifluoromethyl, C₁₋₄ alkoxy, phenoxy and trifluoromethoxy,
pyridinyl, pyridyl, pyrazolyl, imidazolyl, pyrrolidino, wherein the pyridinyl and the pyridyl and the pyrazolyl and the imidazolyl and the pyrrolidino are unsubstituted or substitutued by 1, 2 or 3 substituents selected from the group consisting of C₁₋₄ alkyl, F, Cl, trifluoromethyl, C₁₋₄ alkoxy and trifluoromethoxy,
C₁₋₄ alkoxy, the C₁₋₄ alkoxy being unsubstituted or substituted by 1, 2 or 3 substituents selected from the group consisting of C₁₋₂ alkoxy, F and Cl,
phenoxy,
S(O)ₘ₂R41, C(O)R41 and CO₂R41;
R41 is selected from the group consisting of
N(R50)R51,
linear, branched and cyclic C₁₋₁₀ alkyl, wherein the linear, branched and cyclic C₁₋₁₀ alkyl can be unsubstituted or substituted by 1, 2 or 3 substituents selected from the group consisting of F, Cl, Br, C₁₋₄ alkoxy, C₁₋₄ alkoxy substituted by F, Cl, difluoro, dichloro, bromo or dibromo, carbomethoxy, carboethoxy and OH,
phenyl, naphthyl, wherein the phenyl and the napthyl are unsubstituted or substituted by 1, 2 or 3 substituents selected from the group consisting of C₁₋₄ alkyl, F, Cl, trifluoromethyl, C₁₋₄ alkoxy, phenoxy and trifluoromethoxy,
pyridinyl, pyridyl, pyrazolyl, imidazolyl, pyrrolidino, wherein the pyridinyl and pyridyl and the pyrazolyl and the imidazolyl and the pyrrolidino are unsubstituted or substitutued by 1, 2 or 3 substituents selected from the group consisting of C₁₋₄ alkyl, F, Cl, trifluoromethyl, C₁₋₄ alkoxy and trifluoromethoxy,
m2 is 0, 1 or 2;
R50 and R51 are identical or different and selected from the group consisting of H, C₁₋₂ alkyl, C₁₋₄ alkoxycarbonyl and benzoyl;
- m1: is 0, 1, 2 or 3;
when m1 is 2 or 3, then the substituents R32 can be identical or different from each other; wherein ST1, ST2, R1 and R2 are as defined herein, also with all their embodiments;
further subject of the invention is a method for the preparation of compound of formula (VII), wherein the method comprises the two steps ST1 and ST2, and comprises the two steps ST3 and ST4;
wherein ST3and ST4 are as defined herein, also with all their embodiments;
also with any individual embodiment or with any combination of two or more embodiments,
the embodiments as described herein for any of these steps;
especially wherein
- R1: is phenyl;
- R2: is methyl;
- R31: is tert-butoxy;
- m1: is 0, that is no substituent R32.

Preferably, the preparation of compound of formula (VII) starting from compound of formula (IV) has three steps, a step ST5, a step ST6 and a step ST7, details for these three steps are disclosed in EP 2546236 A1.

ST5 comprises a reaction REAC5, in REAC5 the compound of formula (IV) is reacted with NH₂OH. The reaction product of REAC5 is a compound of formula (V); 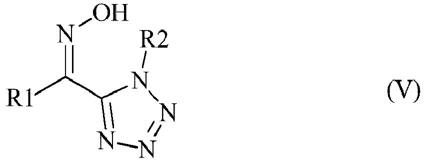 with R1 and R2 as defined above, also with all their embodiments.

ST6 comprises a reaction REAC6, in REAC6 the compound of formula (V) is reacted with a compound of formula (HETLIG); 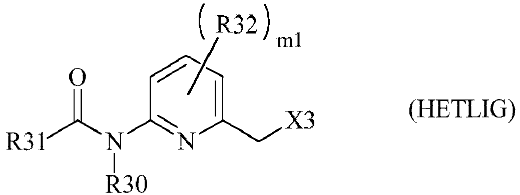
- R30: is selected from the group consisting of H-C(O) and R40-C(O);
- R40: is selected from the group consisting of C₁₋₈ alkyl, C₁₋₆ alkoxy, phenyl, phenyloxy, and pyridyl;
- R40: can be unsubstituted or substituted by 1, 2 or 3 substitutents selected from the group consisting of CN, NO₂, F, Cl, Br, C₁₋₂ alkyl and C₁₋₂ alkoxy;

- X3: is F, Cl, Br or I;
wherein R31, R32 and m1 are as defined herein, also with all their embodiments.

Preferably,
- R30: is selected from the group consisting of methoxycarbonyl, ethoxycarbonyl, isopropoxycarbonyl, n-propoxycarbonyl, n-butoxycarbonyl, iso-butoxycarbonyl, sec-butoxycarbonyl, tert-butoxycarbonyl, formyl, acetyl, n-propylcarbonyl, iso-propylcarbonyl, n-butylcarbonyl, iso-butylcarbonyl, sec-butylcarbonyl, pivaloyl, octanoyl, benzoyl, 2,6-dimethoxybenzoyl, 3,5-nitrobenzoyl, 2,4,6-trichlorobenzoyl, 4-chlorobenzoyl and 2-pyridylcarbonyl;
- R31: is selected from the group consisting of methyl, ethyl, n-propyl, iso-propyl, n-butyl, iso-butyl, sec-butyl, tert-butyl, n-pentyl, n-octyl, cyclopropyl, cyclobutyl, cyclopentyl, cyclohexyl, cycloheptyl, 2,2-dimethylcyclopropyl, menthyl, chloromethyl, fluoromethyl, trifluoromethyl, methoxymethyl, ethoxymethyl, methoxyethyl, methoxypropyl, ethoxybutyl, methoxybutyl, methoxyhexyl, propoxyoctyl, 2-methoxy-1,1-dimethylethyl, 1-ethoxy-1-methylethyl, carbomethoxymethyl, 1-carboethoxy-2,2-dimethyl-3-cyclopropyl, hydroxymethyl, hydroxyethyl, 1-hydroxypropyl, methoxy, ethoxy, n-propoxy, iso-propoxy, n-butoxy, iso-butoxy, sec-butoxy, tert-butoxy, n-pentyloxy, n-hexyloxy, n-decyloxy, cyclopropyloxy, cyclobutyloxy, cyclopentyloxy, cyclohexyloxy, menthyloxy, chloromethoxy, fluoromethoxy, trifluoromethoxy, methoxymethoxy, ethoxymethoxy, methoxyethoxy, 3-ethoxypropoxy, 2-ethoxybutoxy, 4-butoxybutoxy, 1-butoxypentoxy, fluoromethoxymethoxy, dichloromethoxymethoxy, 1 ,2-dibromo-3-methoxypropoxy and 3-isopropoxy-2-methylpropoxy;
- R32: is selected from the group consisting of F, Cl, Br, CN, NO₂, OH, SH, C(O)H, COOH, NH₂, methylamino, dimethylamino, methylethylamino, diethylamino, tert-butoxycarbonylmethylamino, tert-butoxycarbonylamino, acetylmethylamino, acetylethylamino, benzoylmethylamino, methyl, ethyl, n-propyl, iso-propyl, n-butyl, iso-butyl, sec-butyl, tert-butyl, n-pentyl, n-octyl, cyclopropyl, cyclobutyl, cyclopentyl, cyclohexyl, cycloheptyl, 2,2-dimethylcyclopropyl, menthyl, chloromethyl, fluoromethyl, trifluoromethyl, methoxymethyl, ethoxymethyl, methoxyethyl, methoxypropyl, ethoxybutyl, methoxybutyl, methoxyhexyl, propoxyoctyl, 2-methoxy-1,1-dimethylethyl, 1-ethoxy-1-methylethyl, carbomethoxymethyl, 1-carboethoxy-2,2-dimethyl-3-cyclopropyl, hydroxymethyl, hydroxyethyl, 1-hydroxypropyl, phenyl, 1-naphthyl, 2-naphthyl, 6-methylphenyl, 4-methylphenyl, 4-fluorophenyl, 4-chlorophenyl, 2,4-dichlorophenyl, 3,4-dichlorophenyl, 3,5-dichlorophenyl, 2,6-difluorophenyl, 4-trifluoromethylphenyl, 4-methoxyphenyl, 3,4-dimethoxyphenyl, 3-phenoxyphenyl, 4-trifluoromethoxyphenyl, 4-methoxy-1-naphthyl, pyridinyl, pyridyl, pyrazolyl, imidazolyl, pyrrolidino, 3-trifluoromethylpyridin-2-yl, 4-trifluoromethoxy-2-pyridyl, 3-methyl-1-pyrazolyl, 4-trifluoromethyl-1-imidazolyl, 3,4-difluoropyrrolidino, methoxy, ethoxy, n-propoxy, iso-propoxy, n-butoxy, sec-butoxy, iso-butoxy, tert-butoxy, methoxymethoxy, ethoxymethoxy, methoxyethoxy, ethoxyethoxy, phenoxy, trichloromethoxy, trifluoromethoxy, difluoromethoxy, 2,2,2-trifluor-ethoxy and 2-fluoroethoxy,
S(O)ₘ₂R41, C(O)R41 and CO₂R41;
- R41: is selected from the group consisting of NH₂, methylamino, dimethylamino, methylethylamino, diethylamino, tert-butoxycarbonylmethylamino, tert-butoxycarbonylamino, acetylmethylamino, acetylethylamino, benzoylmethylamino, methyl, ethyl, n-propyl, iso-propyl, n-butyl, iso-butyl, sec-butyl, tert-butyl, n-pentyl, n-octyl, cyclopropyl, cyclobutyl, cyclopentyl, cyclohexyl, cycloheptyl, 2,2-dimethylcyclopropyl, menthyl, chloromethyl, fluoromethyl, trifluoromethyl, methoxymethyl, ethoxymethyl, methoxyethyl, methoxypropyl, ethoxybutyl, methoxybutyl, methoxyhexyl, propoxyoctyl, 2-methoxy-1,1 -dimethylethyl, 1-ethoxy-1-methylethyl, carbomethoxymethyl, 1-carboethoxy-2,2-dimethyl-3-cyclopropyl, hydroxymethyl, hydroxyethyl, 1-hydroxypropyl, phenyl, 1-naphthyl, 2-naphthyl, 6-methylphenyl, 4-methylphenyl, 4-fluorophenyl, 4-chlorophenyl, 2,4-dichlorophenyl, 3,4-dichlorophenyl, 3,5-dichlorophenyl, 2,6-difluorophenyl, 4-trifluoromethylphenyl, 4-methoxyphenyl, 3,4-dimethoxyphenyl, 3-phenoxyphenyl, 4-trifluoromethoxyphenyl, 4-methoxy-1-naphthyl, pyridinyl, pyridyl, pyrazolyl, imidazolyl, pyrrolidino, 3-trifluoromethylpyridin-2-yl, 4-trifluoromethoxy-2-pyridyl, 3-methyl-1-pyrazolyl, 4-trifluoromethyl-1-imidazolyl and 3,4-difluoropyrrolidino,
- m2: is 0, 1 or 2;

- m1: is 0, 1 or 2;
- X3: is F, Cl or Br.

More preferably,
- R30: is selected from the group consisting of benzoyl, 2,6-dimethoxybenzoyl, 3,5-nitrobenzoyl, 2,4,6-trichlorobenzoyl and 4-chlorobenzoyl;
- R31: is tert-butoxy;
- m1: is 0;
- X3: is Cl or Br.

The reaction product of REAC6 is compound of formula (VI).

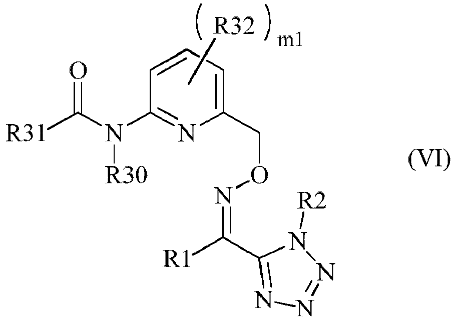

ST7 comprises a reaction REAC7, in REAC7 the compound of formula (VI) is reacted with a base BAS7.

Preferably, BAS7 is selected from the group consisting of alkali metal hydroxides, alkaline earth metal hydroxides, carbonates of alkali metal or of alkaline earth metal, hydrides of alkali metal or of alkaline earth metal, C₁₋₂ alkoxides of alkali metal or of alkaline earth metal, triethylamine, diisopropylethylamine, pyridine, N,Ndimethylaminopyridine, 1,4-diazabicyclo[2.2.2]octane, 4-(dimethylamino)pyridine, 1,8-diazabicyclo[5.4.0]undec-7-ene, 1,5-diazabicyclo[4.3.0]non-5-ene, and mixtures thereof;
more preferably, BAS7 is selected from the group consisting of sodium hydroxide, potassium hydroxide, magnesium hydroxide, calcium hydroxide, sodium carbonate, potassium carbonate, magnesium carbonate, calcium carbonate, sodium hydride, calcium hydride, sodium methoxide, sodium ethoxide, magnesium methoxide, triethylamine, diisopropylethylamine, pyridine, N,Ndimethylaminopyridine, 1,4-diazabicyclo[2.2.2]octane, 4-(dimethylamino)pyridine, 1,8-diazabicyclo[5.4.0]undec-7-ene, 1,5-diazabicyclo[4.3.0]non-5-ene, and mixtures thereof;
even more preferably, BAS7 is selected from the group consisting of sodium hydroxide, potassium hydroxide, magnesium hydroxide, calcium hydroxide, sodium carbonate, potassium carbonate, magnesium carbonate, calcium carbonate, sodium methoxide, sodium ethoxide, magnesium methoxide, triethylamine, diisopropylethylamine, pyridine, N,Ndimethylaminopyridine, 1,4-diazabicyclo[2.2.2]octane, 4-(dimethylamino)pyridine, 1,8-diazabicyclo[5.4.0]undec-7-ene, 1,5-diazabicyclo[4.3.0]non-5-ene, and mixtures thereof;
especially, BAS7 is selected from the group consisting of sodium hydroxide, potassium hydroxide, sodium carbonate, triethylamine, diisopropylethylamine, pyridine, N,Ndimethylaminopyridine, 1,4-diazabicyclo[2.2.2]octane, 4-(dimethylamino)pyridine, 1,8-diazabicyclo[5.4.0]undec-7-ene, 1,5-diazabicyclo[4.3.0]non-5-ene, and mixtures thereof;
more especially, BAS7 is sodium hydroxide.

The reaction product of REAC7 is a compound of formula (VII).

### Examples

### Methods

1H NMR was done with triisobutylphosphate as internal standard, if not otherwise stated.

### Example 1: N-methyl 2-oxophenylthioacetamide

To a mixture of compound of formula (I-1) (0.117 ml, 1.00 mmol) and pyridine (0.016 ml, 0.20 mmol) at 0 °C was added S₂Cl₂ (0.32 ml, 4.00 mmol), and the resulting mixture was stirred at 0 °C for 6 h. Toluene (2.0 ml) was added, and the solution was added dropwise to a rapidly stirred mixture of water (1.0 ml) and an aqueous solution of methyl amine (40% by weight, 0.70 ml, 8.0 mmol) at 0 °C. The mixture was then stirred at room temperature for 15.5 h. The mixture was then diluted with IN aqueous hydrochloric acid (10 ml) and ethyl acetate (2 ml), and a part of the ethyl acetate extract was concentrated and the residue analyzed by 1H NMR. The residue consisted mainly of compound of formula (II-1).

¹H NMR (d6-DMSO, 400 MHz) delta = 11.11 (s, br, 1H), 7.91 (d, br, J = 8 Hz, 2H), 7.68 (t, br, J = 8 Hz, 1H), 7.55 (t, br, J = 8 Hz, 2H), 3.18 (d, J = 3 Hz, 3H).

### Example 2: 1-Methyl-5-benzoyltetrazole

To compound of formula (II-1) (90 mg with 80% being compound of formula (II-1), 0.40 mmol), prepared according to example 1, was added thionyl chloride (0.50 ml, 6.9 mmol). The mixture was stirred at room temperature. Analysis by ¹H NMR after 2 h indicated over 90% conversion to compound of formula (III-1).

After stirring for further 40 min the excess thionyl chloride was evaporated off. The residue was redissolved in toluene (1.0 ml) and the resulting solution was added drop wise at 0 °C to a solution of sodium azide (0.19 g, 3.0 mmol) in water (0.5 ml). The resulting mixture was stirred at 0 °C for 3 h, and then at room temperature for 1 h 20 min. Then the mixture was diluted with brine (10 ml), and the product extracted with ethyl acetate. Yield determination by ¹H NMR indicated a yield of 69% of compound of formula (IV-1).

¹H NMR (d₆-DMSO, 400 MHz) delta = 8.29 (d, br, J = 8 Hz, 2H), 7.80 (t, br, J = 8 Hz, 1H), 7.65 (t, br, J = 8 Hz, 2H), 4.31 (s, 3H).

### Example 3: 1-Methyl-5-benzoyltetrazole

To compound of formula (II-1) (90 mg with 80% being compound of formula (II-1), 0.40 mmol), prepared according to example 1, was added toluene (0.8 ml) and then thionyl chloride (0.109 ml, 1.51 mmol). The mixture was stirred at room temperature for 2 h 40 min, and the excess thionyl chloride was evaporated off. The residue was redissolved in toluene (1.0 ml) and the resulting solution was added dropwise at 0 °C to a solution of sodium azide (0.19 g, 3.0 mmol) in water (0.5 ml). The resulting mixture was stirred at 0 °C for 3 h, and then at room temperature for 1 h 20 min. Then the mixture was diluted with brine (10 ml), and the product extracted with ethyl acetate. Yield determination by ¹H NMR indicated a yield of 76% of compound of formula (IV-1).

## Claims

1. Method for the preparation of compound of formula (II); 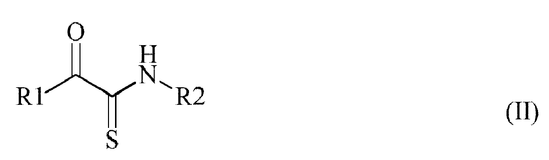 compound of formula (II) is prepared in two steps, a step ST1 and a step ST2;
ST1 comprises a reaction REAC1 of compound of formula (I) with a compound SULFCHLO; SULFCHLO is S₂Cl₂, SCl₂, or a mixture thereof; 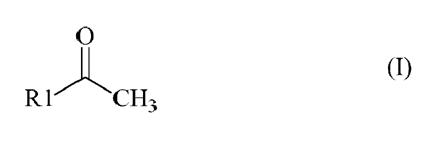 REAC1 results in a reaction product REACPROD1;
ST2 comprises a reaction REAC2 of REACPROD1 with R2-NH₂; REAC2 results in compound of formula (II);
wherein
R1 is selected from the group consisting of tertiary C₄₋₁₂ alkyl, phenyl, the phenyl being unsubstituted or substituted by 1, 2, 3, 4 or 5 substituents selected from the group consisting of halogen, nitro, cyano, trifluoromethyl, C₁₋₈ alkyl, and C₁₋₈ alkoxy, and 5 or 6 membered aromatic heterocycle with 1, 2 or 3 heteroatoms selected from the group consisting of N, O and S, the 5 or 6 membered aromatic heterocycle being unsubstituted or substituted by 1, 2 or 3 substituents selected from the group consisting of halogen, nitro, cyano, trifluoromethyl, C₁₋₈ alkyl, and C₁₋₈ alkoxy;
R2 is selected from the group consisting of C₁₋₁₂ alkyl, the C₁₋₁₂ alkyl being unsubstituted or substituted by 1, 2 or 3 substituents selected from the group consisting of phenyl or 5 or 6 membered aromatic heterocycle with 1, 2 or 3 heteroatoms selected from the group consisting of N, O and S, C₃₋₆ cycloalkyl, phenyl, the phenyl being unsubstituted or substituted by 1, 2, 3, 4 or 5 substituents selected from the group consisting of halogen, nitro, cyano, trifluoromethyl, C₁₋₈ alkyl, and C₁₋₈ alkoxy, and
5 or 6 membered aromatic heterocycle with 1, 2 or 3 heteroatoms selected from the group consisting of N, O and S, the 5 or 6 membered aromatic heterocycle being unsubstituted or substituted by 1, 2 or 3 substituents selected from the group consisting of halogen, nitro, cyano, trifluoromethyl, C₁₋₈ alkyl, and C₁₋₈ alkoxy.

2. Method according to claim 1, wherein
R1 is selected from the group consisting of tertiary C₄₋₁₂ alkyl, phenyl, the phenyl being unsubstituted or substituted by 1, 2 or 3 substituents selected from the group consisting of halogen, nitro, cyano, trifluoromethyl, C₁₋₆ alkyl, and C₁₋₆ alkoxy, and 5 or 6 membered aromatic heterocycle with 1, 2 or 3 heteroatoms selected from the group consisting of N, O and S, the 5 or 6 membered aromatic heterocycle being unsubstituted or substituted by 1, 2 or 3 substituents selected from the group consisting of halogen, nitro, cyano, trifluoromethyl, C₁₋₆ alkyl, and C₁₋₆ alkoxy.

3. Method according to claim 1 or 2, wherein
R2 is selected from the group consisting of C₁₋₆ alkyl, the C₁₋₆ alkyl being unsubstituted or substituted by 1 substituent selected from the group consisting of phenyl or 5 or 6 membered aromatic heterocycle with 1, 2 or 3 heteroatoms selected from the group consisting of N, O and S, C₃₋₆ cycloalkyl, phenyl, the phenyl being unsubstituted or substituted by 1, 2 or 3 substituents selected from the group consisting of halogen, nitro, cyano, trifluoromethyl, C₁₋₆ alkyl, and C₁₋₆ alkoxy, and
5 or 6 membered aromatic heterocycle with 1, 2 or 3 heteroatoms selected from the group consisting of N, O and S, the 5 or 6 membered aromatic heterocycle being unsubstituted or substituted by 1, 2 or 3 substituents selected from the group consisting of halogen, nitro, cyano, trifluoromethyl, C₁₋₆ alkyl, and C₁₋₆ alkoxy.

4. Method according to one or more of claims 1 to 3, wherein
R1 is phenyl; and
R2 is methyl.

5. Method according to one or more of claims 1 to 4, wherein
SULFCHLO is S₂Cl₂.

6. Method according to one or more of claims 1 to 5, wherein
REAC1 is done in the presence of a catalyst, the catalyst is a compound CAT1, CAT1 is selected from the group consisting of pyridine, 2-picoline, 3-picoline, 4-picoline, 5-ethyl-2-methylpyridine, 4-(dimethylamino)pyridine, 1,4-diazabicyclo[2.2.2]octane, triethylamine, tributylamine, N-methylmorpholine, N-ethyldiisopropylamine, N,N-dimethylaniline, and mixtures thereof.

7. Method for the preparation of compound of formula (IV); 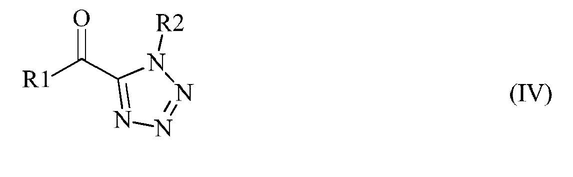 the method comprises four steps, ST1, ST2, a step ST3 and a step ST4;
ST4 comprises a reaction REAC4 of a compound of formula (III) with a compound AZID; 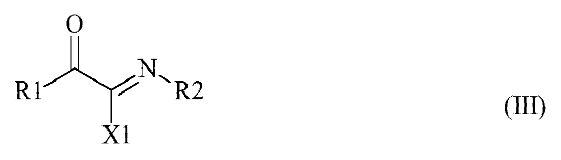 AZID is selected from the group consisting of alkali metal azide, alkali earth metal azide, [N(R10)(R11)(R12)R13]⁺ [N₃]⁻ and guanidinium azide;
R10, R11, R12 and R13 are identical or different and independently from each other selected from the group consisting of H and C₁₋₈ alkyl;
X1 is selected from the group consisting of Cl, Br, S-CH₃, S-CH₂-CH₃, S-CH₂-Phenyl, S-C(O)-C(CH₃)₃, and SO₃H;
REAC4 results in compound of formula (IV);
compound of formula (III) is prepared in ST3;
ST3 comprises a reaction REAC3 of a compound of formula (II) with a compound COMPREAC3;
COMPREAC3 is selected from the group consisting of thionyl chloride, COCl₂, diphosgene, triphosgene, POCl₃, PCl₃, PCl₅, POBr₃, PBr₃, PBr₅, S₂Cl₂, SCl₂, pivaloyl chloride, methyl chloride, methyl bromide, methyl iodide, ethyl chloride, ethyl bromide, ethyl iodide, benzyl chloride, benzyl bromide, dimethyl sulfate, diethyl sulfate, trimethylphosphate, triethylphosphate, methyl methanesulfonate, methyl benzenesulfonate, methyl tosylate, ethyl methanesulfonate, ethyl benzenesulfonate, ethyl tosylate, hydrogen peroxide, C₁₋₄ alkyl hydro peroxide, C₁₋₆ alkaloyl peroxide, perbenzoic acid, 3-chloro-perbenzoic acid, alkali metal persulfate salt, Cl₂, Br₂, and I₂;
REAC3 results in compound of formula (III);
compound of formula (II) is prepared in the two steps ST1 and ST2;
with ST1, ST2, R1 and R2 as defined in any one of claims 1 to 6.

8. Method according to claim 7, wherein
AZID is selected from the group consisting of alkali metal azide, and
[N(R10)(R11)(R12)R13]⁺ [N₃]⁻.

9. Method according to claim 7 or 8, wherein
COMPREAC3 is selected from the group consisting of thionyl chloride, COCl₂, diphosgene, triphosgene, POCl₃, PCl₃, PCl₅, S₂Cl₂, pivaloyl chloride, methyl chloride, methyl bromide, methyl iodide, ethyl chloride, ethyl bromide, ethyl iodide, benzyl chloride, benzyl bromide, dimethyl sulfate, diethyl sulfate, trimethylphosphate, triethylphosphate, methyl methanesulfonate, methyl benzenesulfonate, methyl tosylate, ethyl methanesulfonate, ethyl benzenesulfonate, ethyl tosylate, hydrogen peroxide, C₁₋₄ alkyl hydro peroxide, C₁₋₆ alkaloyl peroxide, perbenzoic acid, 3-chloro-perbenzoic acid, sodium persulfate salt, potassium persulfate salt, Cl₂, Br₂, and I₂.

10. Method according to claim 7 or 8, wherein
X1 is selected from the group consisting of Cl, S-CH₃, S-CH₂-CH₃, S-CH₂-Phenyl, S-C(O)-C(CH₃)₃, and SO₃H.

11. Method for the preparation of compound of formula (VII), 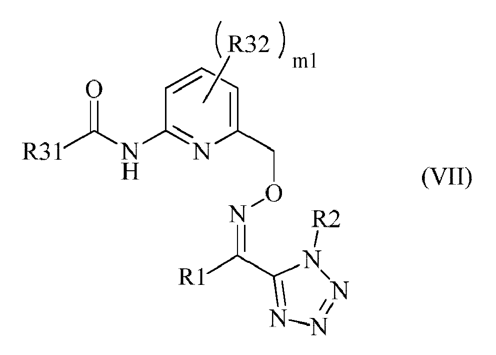 wherein the method comprises the two steps ST1 and ST2 as defined in one or more of claims 1 to 6;
R1 and R2 are as defined in anyone of claims 1 to 4;
R31 is selected from the group consisting of linear, branched and cyclic C₁₋₁₀ alkyl and linear, branched and cyclic C₁₋₁₀ alkoxy, wherein the linear, branched and cyclic C₁₋₁₀ alkyl and the linear, branched and cyclic C₁₋₁₀ alkoxy can be unsubstituted or substituted by 1, 2 or 3 substituents selected from the group consisting of F, Cl, Br, C₁₋₄ alkoxy, C₁₋₄ alkoxy substituted by F, Cl, difluoro, dichloro, bromo or dibromo, carbomethoxy, carboethoxy and OH;
R32 is selected from the group consisting of F, Cl, Br, CN, NO₂, OH, SH, C(O)H, COOH, N(R50)R51, linear, branched and cyclic C₁₋₁₀ alkyl, wherein the linear, branched and cyclic C₁₋₁₀ alkyl can be unsubstituted or substituted by 1, 2 or 3 substituents selected from the group consisting of F, Cl, Br, C₁₋₄ alkoxy, C₁₋₄ alkoxy substituted by F, Cl, difluoro, dichloro, bromo or dibromo, carbomethoxy, carboethoxy and OH, phenyl, naphthyl, wherein the phenyl and the napthyl are unsubstituted or substituted by 1, 2 or 3 substituents selected from the group consisting of C₁₋₄ alkyl, F, Cl, trifluoromethyl, C₁₋₄ alkoxy, phenoxy and trifluoromethoxy, pyridinyl, pyridyl, pyrazolyl, imidazolyl, pyrrolidino, wherein the pyridinyl and the pyridyl and the pyrazolyl and the imidazolyl and the pyrrolidino are unsubstituted or substitutued by 1, 2 or 3 substituents selected from the group consisting of C₁₋₄ alkyl, F, Cl, trifluoromethyl, C₁₋₄ alkoxy and trifluoromethoxy, C₁₋₄ alkoxy, the C₁₋₄ alkoxy being unsubstituted or substituted by 1, 2 or 3 substituents selected from the group consisting of C₁₋₂ alkoxy, F and Cl, phenoxy, S(O)ₘ₂R41, C(O)R41 and CO₂R41; R41 is selected from the group consisting of N(R50)R51, linear, branched and cyclic C₁₋₁₀ alkyl, wherein the linear, branched and cyclic C₁₋₁₀ alkyl can be unsubstituted or substituted by 1, 2 or 3 substituents selected from the group consisting of F, Cl, Br, C₁₋₄ alkoxy, C₁₋₄ alkoxy substituted by F, Cl, difluoro, dichloro, bromo or dibromo, carbomethoxy, carboethoxy and OH, phenyl, naphthyl, wherein the phenyl and the napthyl are unsubstituted or substituted by 1, 2 or 3 substituents selected from the group consisting of C₁₋₄ alkyl, F, Cl, trifluoromethyl, C₁₋₄ alkoxy, phenoxy and trifluoromethoxy, pyridinyl, pyridyl, pyrazolyl, imidazolyl, pyrrolidino, wherein the pyridinyl and pyridyl and the pyrazolyl and the imidazolyl and the pyrrolidino are unsubstituted or substitutued by 1, 2 or 3 substituents selected from the group consisting of C₁₋₄ alkyl, F, Cl, trifluoromethyl, C₁₋₄ alkoxy and trifluoromethoxy, m2 is 0, 1 or 2; R50 and R51 are identical or different and selected from the group consisting of H, C₁₋₂ alkyl, C₁₋₄ alkoxycarbonyl and benzoyl; m1 is 0, 1, 2 or 3;
when m1 is 2 or 3, then the substituents R32 can be identical or different from each other.

12. Method according to claim 11,
wherein the method comprises the two steps ST3 and ST4 as defined in anyone of claims 7 to 10.

13. Method according to claim 11 or 12, wherein
R1 is phenyl;
R2 is methyl;
R31 is tert-butoxy;
m1 is 0.
